(19) Europäisches Patentamt
European Patent Office
Office européen des brevets

(11) **EP 3 223 912 B1**

(12) **EUROPEAN PATENT SPECIFICATION**

(45) Date of publication and mention
of the grant of the patent:
**01.04.2020  Bulletin 2020/14**

(21) Application number: **14821471.1**

(22) Date of filing: **19.12.2014**

(51) Int Cl.:
***A61K 8/85*** *(2006.01)*       ***A61Q 11/00*** *(2006.01)*
***A61K 8/11*** *(2006.01)*

(86) International application number:
**PCT/US2014/071432**

(87) International publication number:
**WO 2016/099543 (23.06.2016 Gazette 2016/25)**

(54) **ORAL CARE COMPOSITIONS CONTAINING BIODEGRADABLE PARTICLES WITH SUPERIOR AESTHETICS**

ZUSAMMENSETZUNGEN FÜR MUNDPFLEGE ENTHALTEND ABBAUBARE PARTIKEL VON GEHOBENER ÀSTHETIK

COMPOSITIONS ORALES CONTENANT LES PARTICULES BIODEGRADABLES D'UNE BEAUTÉ SUPÉRIEURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.10.2017   Bulletin 2017/40**

(73) Proprietor: **Colgate-Palmolive Company New York, NY 10022 (US)**

(72) Inventor: **D'AMBROGIO, Robert Princeton, New Jersey 08540 (US)**

(74) Representative: **Wibbelmann, Jobst Wuesthoff & Wuesthoff Patentanwälte PartG mbB Schweigerstrasse 2 81541 München (DE)**

(56) References cited:
**WO-A1-2006/125661      WO-A1-2009/023393 WO-A2-2008/060778**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Dyes encapsulated in polyethylene have been added to toothpastes, mouthrinse and other oral care compositions to provide colored speckles or other colorful decorative patterns in order to improve consumer acceptability of these products. While polyethylene has been used to encapsulate dyes for use in aesthetically pleasing products, there is growing public concern that such microplastics may contribute to global pollution since they are not readily biodegradable.

Biodegradable polymers, such as polylactic acid, are known in the art. Polylactic acid and its degradation products, namely $H_2O$ and $CO_2$, are neither toxic nor carcinogenic to the human body. Accordingly, this polymer has found use in biomedical applications.

For example, polylactic acid polymers have been reported to be useful carriers for pharmaceuticals such as small molecules and proteins. The degradation properties of these polymers via hydrolysis are beneficial for releasing the pharmaceutical into the patient. However, such degradation characteristics appear to be contraindicated for water containing products where degradation during storage and use is undesirable. Accordingly, there remains a desire in the art to provide aesthetically pleasing colored oral care products to consumers without the use of polyethylene.

**BRIEF SUMMARY**

The present disclosure is directed to a stable oral care composition including an orally acceptable vehicle, and a colorant encapsulated by a biodegradable polylactic acid-based polymer.

Also provided herein is a method of preparing an oral care composition including: (a) mixing water into an orally acceptable vehicle to form a mixture and (b) dispersing a colorant encapsulated in a biodegradable polylactic acid-based polymer into the mixture formed in (a) to form a stable oral care composition.

The invention can be used for promoting oral health by contacting a mouth surface with said stable oral care composition.

**DETAILED DESCRIPTION**

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

Compositions

The present inventor surprisingly recognized that colorants encapsulated by biodegradable polymers may be used to provide aesthetically pleasing stable oral care compositions that do not bleed or leach even when the compositions contain water. As used herein, the term "encapsulated" means to surround particles or droplets of the colorant with a biodegradable polymer to provide microcapsules ranging in size from e.g., about 10 $\mu$m to about 1000 $\mu$m, more typically about 100 $\mu$m to about 500 $\mu$m, even more typically about 100 $\mu$m to about 300 $\mu$m, yet even more typically about 230 $\mu$m. The term "microcapsule" refers to a microparticle including, for example, a biodegradable polymeric or copolymeric shell serving as a wall-forming material and an encapsulated material, *e.g.*, a colorant, located within the core of the microcapsule. As used herein, "microcapsules" also encompass homogeneous granules of the colorant dispersed in the described polymer lacking a distinctive external wall phase. Microcapsules may be any shape, *e.g.* spherical, other geometric shaped or irregularly shaped.

As described herein, the present encapsulated colorants dispersed within the present stable oral care composition demonstrate minimal migration into a vehicle of the stable oral care composition. Accordingly, the colorant minimally reacts with the other ingredients of the composition and the integrity of the color of the colorant is maintained. For example, the colorant does not fade or change color as may occur upon contact with a component in the vehicle. Further, the colorant is able to be dispersed as discrete particles, such as speckles or other patterns within the vehicle of the stable oral care composition without bleeding or leaching into the vehicle.

*Biodegradable polylactic acid-based polymer*

According to the invention, the colorant is encapsulated in a biodegradable polylactic acid-based polymer, which may have a melting point between about 80°C to about 180°C, more typically about 130 °C to about 150 °C, even more typically ranging from about 140°C to about 150°C, is capable of forming fine droplets when sprayed from a spray nozzle, is a solid at room temperature and liquefies without destruction upon heating. The biodegradable polylactic acid-based polymer is stable when in contact with water or other ingredients, such as flavors, glycerin, sorbitol, surfactants and other materials, which are standardly present in oral care compositions. In some embodiments, the polymer is one which can be dispersed in suitable solutes or creams, *etc.*, having been made into, for example, a microcapsule or microsphere.

The term "biodegradable" as used herein refers to polymers containing chemical linkages that can be broken down by hydrolysis, enzymes and/or bacteria to form a lower molecular weight species. The biodegradable polylactic acid-based polymers described in the present disclosure encompass those that meet the biodegradability criteria specified in ASTM 6400. More particularly, the ASTM 6400 criteria are designed to test the degree and rate of aerobic biodegradation of plastic materials on exposure to a controlled composting environment under laboratory conditions. The test is intended to mimic recognized composting conditions in municipal and industrial aerobic composting facilities. The parameters used are those required to determine if the plastics and products made from the plastics will compost satisfactorily, including biodegrading at a rate comparable to known compostable materials. Further, the properties in the ASTM 6400 specification are required to assure that the degradation of these materials will not diminish the value or utility of the compost resulting from the composting process. For example, ASTM 6400 criteria state that satisfactory disintegration of a test material is indicated when after twelve weeks in a controlled composting environment, no more than 10% of the test material's original dry weight remains after sieving on a 2.0-mm sieve. Further, at least 60% of the organic carbon must be converted to carbon dioxide by the end of the test period, when compared to a positive control such as cellulose. In addition, no adverse effects on the quality of the compost should be observed. For example, the germination rate and the plant biomass of the sample composts should be no less than 90 % to that of corresponding control composts for two different plant species. Further, heavy metals must meet acceptable levels as indicated by the ASTM 6400 guidelines.

The term "polymer" as used herein, is given its ordinary meaning as used in the art, *i.e.*, a molecular structure comprising one or more repeat units (monomers), connected by covalent bonds. The repeat units may all be identical, or in some cases, there may be more than one type of repeat unit present within the polymer. If more than one type of repeat unit is present within the polymer, then the polymer is said to be a "copolymer." The repeat units forming the copolymer may be arranged in any fashion. For example, the repeat units may be arranged in a random order, in an alternating order, or as a block copolymer, *i.e.*, comprising one or more regions each comprising a first repeat unit (e.g., a first block), and one or more regions each comprising a second repeat unit (e.g., a second block), *etc.* Block copolymers may have two (a diblock copolymer), three (a triblock copolymer), or more numbers of distinct blocks.

Biodegradable polylactic acid-based polymers used to encapsulate the colorants of the present disclosure include, for example, polylactic acid, poly(lactide-co-glycolide) polymer (PLGA), and copolymers, derivatives, and mixtures thereof.

According to the invention, polylactic acid-based polymers are used as the encapsulating material. As used herein, a "polylactic acid-based polymer" is a homopolymer or a copolymer having at least about 20% or more by weight of polylactic acid. In some embodiments, the polylactic acid-based polymer has at least about 50%, at least about 60% at least about 75%, at least about 80% or at least about 90% by weight of polylactic acid.

The term "polylactic acid," includes any one or more of four morphologically distinct polylactic acid polymers: D-polylactic acid, L-polylactic acid, D, L-polylactic acid, and meso-polylactic acid. "D-polylactic acid" and "L-polylactic acid" are dextro-polylactic acid and levo-polylactic acid, respectively, and both of them are optically active polymers that rotate a light vector when transmitted through the polymer. "D, L-polylactic acid" is a racemic polymer, *i.e.*, a copolymer of D-polylactic acid and L-polylactic acid having a well-defined conformation of D-and L-polylactic acid units. "Meso-polylactic" is a random copolymer of D-polylactic and L-polylactic.

In some embodiments, suitable biodegradable polylactic acid-based polymers of the present disclosure include copolymers comprising monomers such as ε-caprolactone glycolide *p*-dioxanone, valeric acid or butyric acid. In some embodiments, the polylactic acid-based polymers of the present disclosure are blended with or copolymerized with another biodegradable polymer. Such suitable biodegradable polymers include but are not limited to polycaprolactone, polyglycolic acid, poly(lactide-co-glycolide) polymer (PLGA), and copolymers, derivatives, and mixtures thereof, polyanhydrides, poly(ortho)esters, polyurethanes, poly(butyric acid), poly(valeric acid), poly(lactide-co-caprolactone), blends, copolymers and derivatives thereof. As used herein, "derivatives" include polymers having substitutions, additions of chemical groups and other modifications routinely made by those skilled in the art.

In some embodiments, the polylactide polymer has a weight average molecular weight (Mw) ranging from about 100,000 to about 500,000 daltons. In other embodiments, the weight average ranges from 100,000 to 300,000 daltons, and in yet other embodiments ranges from 100,000 to about 250,000 daltons.

In some embodiments, the polylactic acid polymer is a copolymer having a 75:25 weight ratio of polylactic acid(PLA): polyglycolide (PGA) with a molecular weight of 76,000-116,000 daltons. In other embodiments, the polylactic acid polymer is a copolymer having an 85:15 weight ratio PLA:PGA with a molecular weight of 190,000-240,000 daltons.

Methods for preparing polylactic acid polymers are well known in the art. For example, polylactic acid polymers may be prepared by polycondensation (including solution polycondensation and melt polycondensation) of for example D- or L-lactic acid or combinations thereof as described herein, more typically, from ring opening polymerization of lactide, a cyclic dimer of lactic acid. Chemical modification of polylactic acid polymers may be performed by copolymerizing lactic acid with other monomers or by crosslinking or adding organic or inorganic filler/fiber materials to modify physical characteristics and/or reduce overall material cost.

Non-limiting examples of organic fillers include wood flour, seeds, polymeric particles, ungelatinized starch granules, cork, gelatins, saw dust, milled polymeric materials, agar-based materials, and the like.

Examples of inorganic fillers include calcium carbonate, titanium dioxide, silica, talc, mica, sand, gravel, crushed rock, bauxite, granite, limestone, sandstone, glass beads, aerogels, xerogels, clay, alumina, kaolin, microspheres, hollow glass spheres, porous ceramic spheres, gypsum dihydrate, insoluble salts, magnesium carbonate, calcium hydroxide, calcium aluminate, magnesium carbonate, ceramic materials, pozzolanic materials, salts, zirconium compounds, xonotlite (a crystalline calcium silicate gel), lightweight expanded clays, perlite, vermiculite, hydrated or unhydrated hydraulic cement particles, pumice, zeolites, exfoliated rock, ores, minerals, and the like. In some embodiments, 10 wt% to 30 wt%, such as 15-30%, such as 15% to 25% of organic fillers, inorganic fillers or fibers are used in the biodegradable polymers of the present disclosure.

Non-limiting examples of fibers that may be incorporated into the biodegradable compositions include naturally occurring organic fibers, such as cellulosic fibers extracted from wood, plant leaves, and plant stems. These organic fibers can be derived from cotton, wood fibers (both hardwood or softwood fibers, examples of which include southern hardwood and southern pine), flax, abaca, sisal, ramie, hemp, and bagasse. In addition, inorganic fibers made from glass, graphite, silica, ceramic, rock wool, or metal materials may also be used.

Suitable commercially available polylactic acid polymers may be obtained from NatureWorks™ from Cargill Dow, USA, LACEA from Mitsui Chemicals, Japan, and Lacty from Shimadzu Seisakusho, Japan for example. In certain embodiments, polylactic acid polymers include PLA NatureWorks™ 5729B, 2002D, and 5040D available from Cargill Dow.

*Colorants*

As noted above, the biodegradable polymers described herein encapsulate a colorant. As used herein, the term "colorant" refers to a substance in the form of a dry powder or liquid that imparts color to another substance. Generally, colorants include dyes, lakes, pigments or combinations thereof. In some embodiments, mica, mica-based pearlescent pigments, talc, calcium carbonate and silica may also be used as colorants. More typically, the colorant is a dye, lake or a combination of one or more dyes and/or one or more lakes.

As used herein, the term "dye" refers to an organic species, which is essentially water soluble in an aqueous medium in which the dye remains chemically stable. "Lakes" are manufactured by precipitating a dye with an inert binder, or "mordant", usually a metallic salt. Salts that are commonly used include barium sulfate, calcium sulfate, aluminum hydroxide, and aluminum oxide (alumina). In some embodiments, aluminum lakes are used.

A "pigment" is a synthetic or natural water insoluble substance, which imparts color to another substance.

The dyes used with the stable oral care compositions of the present disclosure are generally food color additives presently certified under the Food Drug & Cosmetic Act for use in food and ingested drugs, including dyes such as FD&C Blue No. 1, FD&C Blue No. 2, FD&C Green No. 3, D&C Green No. 5, D&C Orange No. 5, FD&C Red No. 3, D&C Red No. 21, D&C Red No. 22, D&C Red No. 27, D&C Red No. 28, D&C Red No. 30, D&C Red No. 40, FD&C Yellow No. 5, FD& C Yellow No. 6 and D&C Yellow No. 10 and mixtures thereof in various proportions.

Lakes which may be used with the present stable oral compositions of the present disclosure include but are not limited to FD&C Blue No. 1 Aluminum Lake, FD&C Blue No. 2 Aluminum Lake, FD&C Green No. 3 Aluminum Lake, FD&C Red No. 3 Aluminum Lake, FD&C Red No. 40 Aluminum Lake, FD&C Yellow No. 5 Aluminum Lake, FD& C Yellow No. 6 Aluminum Lake. In certain embodiments the colorant is selected from FD&C Blue No. 1 Aluminum Lake, FD&C Yellow No. 5 Aluminum Lake, and FD&C Red No. 40 Aluminum Lake.

In some embodiments, the colorants are pigments. Pigments include non-toxic, water insoluble inorganic pigments such as titanium dioxide and chromium oxide greens, ultramarine blues and pinks and ferric oxides, *e.g.*, annatto extract, chlorophyllin-copper complex, canthaxanthin, β-carotene, synthetic iron oxide, $TiO_2$ and mixtures thereof.

Suitable dyes and lakes, their structures, and properties for use herein are well known to those skilled in the art, *see*, *for example*, Kirk-Othmer Encyclopedia of Chemical Technology, 3rd Edition, Volume 6, pg. 561-596.

In some embodiments, the compositions are stable. As used herein "stable" means that migration of the colorant into the vehicle of the present oral care composition is suppressed, mitigated or prevented by the biodegradable coating for a period of time, such that a reaction of the colorant with the other ingredients of the composition resulting in a change in the colorant, for example fading of the colorant, is not visually observed. "Stable" also refers to the lack or suppression of ability or the lack or suppression of propensity of the encapsulated colorant to leach or bleed or migrate into the vehicle of the present stable dentifrice composition, such that the integrity of discrete particles or patterns of the encapsulated colorant in the vehicle may be maintained when visually observed. As used herein "not visually observed" or "not visually observable" means that the colorant encapsulated in the biodegradable polymer is not visualized or observable as bleeding or leaching into the vehicle or that fading or a color change of the colorant is not visualized or observable when viewed with the naked eye.

In various embodiments, migration of colorant encapsulated in the biodegradable polymer is suppressed and not visually observed at temperatures ranging from about 20°C to about 50°C, for example from about 20°C to about 40°C or about 30°C or about 40°C.

In some embodiments, migration of the colorant into the vehicle is suppressed and not visually observable for a duration of time or a period of time, such as about one day, about one week, about one month, about two months, about three months, about four months, about six months, about one year, about two years or about three years or more.

The term "stable" as used herein also encompasses migration of a specified amount of the colorant into the present vehicle of the oral care composition of the instant disclosure where the amount is about 0%, less than about 1%, less than about 2%, less than about 3%, less than about 4%, less than about 5%, less than about 10% or less than about 20% at temperatures ranging from about 20°C to about 50°C, for example from about 20°C to about 40°C or about 30°C or about 40°C, for a specified duration of time, where the duration is about one day, about one week, about one month, about two months, about three months, about four months, about six months, about one year, about two years or about three years or more.

Colorants that are encapsulated in a biodegradable polymer and are suitable for use with the present stable oral care composition are commercially available. For example, FD & C Blue No. 1 Lake surrounded by a polylactic acid protective coating (EcoBlue 50BL1) may be purchased from Micro Powders, Inc., Tarrytown, NY.

In some embodiments, colorants encapsulated in a biodegradable polymer may be prepared using methods well known in the art including but not limited to spray drying, interfacial polymerization, hot melt encapsulation, phase separation encapsulation (solvent removal and solvent evaporation), spontaneous emulsion and coacervation, *see, for example,* Kirk-Othmer Encyclopedia of Chemical Technology, Third Edition, Vol. 125 Pgs. 470-493 (1981).

An example of a method by which colorants, such as lakes or dyes of the present disclosure, may be encapsulated in a polymer, such as polylactic acid, is by dispersing the desired amount of colorant in the polymer that has been thermally softened to form a liquid composition. The ratio of lake or dye, for example, to a polymer, such as polylactic acid, ranges from about 0.1% to about 9%, more typically about 0.1% to about 6%, even more typically about 0.1-3%. In some embodiments, the ratio of a pigment colorant to a biodegradable polymer or the ratio of mica, talc, calcium carbonate and silica to a biodegradable polymer, such as polylactic acid, ranges from 1% to 50%, more typically 10% to 50% or even more typically 30% to 50%.

The desired amount of colorant, in some embodiments, is the amount of colorant which results in a final concentration of up to 20% colorant in the present oral care compositions, for example, about 0.1% to about 12%, about 0.1% to about 10%, about 3% to about 10%, about 0.1 to about 5%, about 0.1% to about 2% or about 0.1% to about 1% by weight.

The liquid polymer dispersion may be agitated so that the liquid polymer deposits on each entity of the colorant material to form liquid polymer walled droplets. The dispersion is then cooled and milled to provide solid particles in which the colorant, for example, the dye or lake is encapsulated. The particle size distribution of the polymer particles can vary from about 10 μm to about 1000 μm.

*Orally Acceptable Vehicle*

The encapsulated colorants as described herein are combined with an orally acceptable vehicle to form an oral care composition. An "oral care composition" as used herein refers to a composition, which is retained in the oral cavity for a time sufficient to contact substantially all of the dental surfaces and/or oral tissues for purposes of oral activity. The oral care composition of the present invention may be in various forms including mouthrinses or dentifrices. Dentifrices may include a dental tablet, toothpaste (dental cream), tooth powders, gel, liquid, liquigel, or any other form known to one of skill in the art.

As used herein, an "orally acceptable vehicle" refers to a material or combination of materials that are safe for use in the compositions of the present disclosure, commensurate with a reasonable benefit/risk ratio, with which any desired active ingredients may be associated while retaining significant efficacy.

In some embodiments, the orally acceptable vehicle is a high water content orally acceptable vehicle and may include any known ingredients or additives. For example, the vehicle may include liquid mixtures of water, glycerin, sorbitol and sodium chloride. In such embodiments, water is present typically in amount of at least about 20% by weight, and generally about 25 to 50% by weight, more typically about 40% to 75%, even more typically ranging from 77% to 88% by weight of the oral composition. Water employed in the preparation of commercially suitable oral compositions should preferably be deionized and free of organic impurities.

In some embodiments, the orally acceptable vehicle includes structurants, such as for use in a structured mouthrinse, *i.e.* a mouthrinse that includes a material that provides rheological and structuring benefits, for example as measured by static yield stress. Such structurants include but are not limited to polymers or gum structurants that may swell or expand when hydrated to form random dispersion of independent microgel particles. Examples of polymers and gums structurants include: gellan gum, pectin, alginate, arabinogalactan, caageenan, xanthum gum, guar gum, rhamsan gum, furcellaran gum, carboxymethylcellulose and cellulose. *See also* PCT Publication No. WO 2014088534. In some embodiments, the structuring agents can be used to provide a static yield stress of from 0.20 to 400 Pa.

In some embodiments, the orally acceptable vehicle comprises 0.05 to 1.00 weight % xanthan gum. In certain embodiments, particularly when the orally acceptable vehicle is used as a mouthrinse, the vehicle comprises 0.01 wt% to 0.25 wt% xanthan gum, 0.01 wt% to 0.30 wt% low acyl gellan gum or a combination thereof.

In some embodiments, the vehicle is a low water content orally acceptable vehicle and may include any known ingredients or additives. For example, the vehicle may include liquid mixtures of water, glycerin, and sorbitol. In some embodiments, the water content of the stable oral composition is less than about 20%, less than about 10%, less than about 8% less than about 4%, less than about 2%, less than about 1% or less than about 0.1% w/w.

In some embodiments, the orally acceptable carrier is substantially anhydrous. Suitable low humectants/vehicles for such embodiments include but are not limited to polyethylene glycol, such as PEG400, PEG600, PEG/PPG copolymers, such as PEG/PPG 38/8 copolymer, PEG/PPG-1 16/66 copolymer sold as PLURACARE® L4370 and PLURACARE® L1220 from BASF corporation of Wyandotte, Michigan, respectively. In some embodiments, the humectant/vehicle is present in a total amount of from 2% to 55% w/w and typically from 2% to 35% w/w. Propylene glycol or glycerin may also be present in an amount from 0% to 15% w/w.

In some embodiments, the viscosity of the oral care composition ranges from 200 cps to 1,000,000 cps. For example, the viscosity of a mouthrinse may range from 200 to 1000 cps, a liquigel toothpaste from about 5,000 to 60,000 cps and a dentifrice from about 60,000 cps to 1,000,000 cps.

In some embodiments, surfactants may be added to enhance stability of the formulation, help clean the oral cavity surfaces through detergency, and provide foam upon agitation, e.g., during brushing or rinsing with an oral care composition of the disclosure. Surface active agents may achieve increased prophylactic action, by thoroughly dispersing an anti-calculus agent, for example, throughout the oral cavity. In various embodiments, suitable surface active agents may function as a surface active agent, emulsifier, and/or foam modulator.

Any orally acceptable surfactant, most of which are anionic, nonionic or amphoteric, can be used. Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers such as polyoxomer 407, polyoxyethylene sorbitan esters such as polysorbate 20, fatty alcohol ethoxylates, alkylphenol ethoxylates, tertiary amine oxides, tertiary phosphine oxides, dialkyl sulfoxides and the like. Suitable amphoteric surfactants include without limitation derivatives of $C_{8-20}$ aliphatic secondary and tertiary amines having an anionic group such as carboxylate, sulfate, sulfonate, phosphate or phosphonate. A suitable example is cocoamidopropyl betaine.

In some embodiments, one or more surfactants may be present in a total amount of from about 1.8% to about 2% w/w. In some embodiments, one or more surfactants may be present in a total amount of from about 1.9% to about 2% w/w. In some embodiments, one or more surfactants may be present in a total amount of about 2% w/w.

In some embodiments, the composition optionally comprises a thickening agent. Any orally acceptable thickening agent can be used, including without limitation carbomers, also known as carboxyvinyl polymers, carrageenans, also known as Irish moss and more particularly -carrageenan (iota-carrageenan), high molecular weight polyethylene glycols (such as CARBOWAX, available from The Dow Chemical Company), cellulosic polymers such as hydroxyethylcellulose, carboxymethylcellulose (CMC) and salts thereof, *e.g.*, CMC sodium, natural gums such as low acyl gellan, xanthan, karaya gum arabic and tragacanth, colloidal magnesium aluminum silicate, and colloidal and/or thickener silica and mixtures of the same.

In some embodiments, a thickener silica is used with the present orally acceptable vehicle. In certain embodiments, the thickener silica is a synthetic amorphous precipitated material of high surface area and internal pore volume to provide water absorption of about 50 ml or greater / 20 grams of silica and oil absorption of about 200 ml or greater /100g silica (per ASTM D281 method). Examples of thickener silicas which may be used are Zeodent® 165, Zeodent® 163 and Zeodent® 153 (from Huber); Aerosil® 200 and Sident® 22S (from Evonik); Sylodent® 15 and Perkasil® SM 660 (from W.R. Grace & Co.); and Tixocil 43B (From Rhodia).

In some embodiments, the one or more optional thickening agents are present in a total amount of about 0.01 % to about 90% w/w. In some embodiments, the one or more optional thickening agents are present in a total amount of about 1% to about 50% w/w. In some embodiments, the one or more optional thickening agents are present in a total amount of about 5% to about 35% w/w.

In some embodiments, particularly when the orally acceptable vehicle is used as a dentifrice, such as a liquigel toothpaste, the vehicle comprises 0.50 wt% to 10 wt% silica.

In certain embodiments, particularly mouthrinse embodiments, the orally acceptable vehicle comprises 0.10 wt% to 1.0 wt% monovalent or divalent chloride salt such as $CaCl_2$, NaCl or a mixture thereof, to attain a viscosity ranging from 200 to 1000 cps, for example.

Useful flavoring agents, which may be used with an orally acceptable vehicle of the present disclosure, include any material or mixture of materials operable to enhance the taste of the composition. Any orally acceptable natural or synthetic flavoring agent can be used, such as flavoring oils, flavoring aldehydes, esters, alcohols, similar materials, and combinations thereof. Flavoring agents include vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants, and mixtures thereof. Food-grade phosphoric acid may be used to acidify the oral care compositions of the present disclosure and provide a tangy and sour taste. Also encompassed within flavoring agents herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, x-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methone glycerol acetal (MGA) and mixtures thereof.

In some embodiments, one or more flavoring agents are optionally present in a total amount of about 0.01% to about 5% w/w. In some embodiments, one or more flavoring agents are optionally present in a total amount of about 0.05% to about 2% w/w. In some embodiments, one or more flavoring agents are optionally present in a total amount of about 0.1% to about 2.5% w/w. In some embodiments, one or more flavoring agents are optionally present in a total amount from about 0.1 % to about 0.5% w/w. In some embodiments, one or more flavoring agents are optionally present in the total amount of about 2.25% w/w.

Sweeteners among those useful herein include orally acceptable natural or artificial, nutritive or non-nutritive sweeteners. Such sweeteners include dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones and mixtures thereof. Some embodiments optionally comprise one or more sweeteners. In some embodiments, the one or more optional sweeteners are present in a total amount from about 0.005% to about 5% w/w. In some embodiments, the one or more optional sweeteners are present in a total amount from about 0.01 to about 1 % w/w.

Suitable preservatives such as sodium benzoate and may be present in the orally acceptable vehicle in an amount based upon the total weight of the composition, for example, from about 0% to about 0.2%, and typically from about 0.05% to about 0.10%.

*Active Ingredients*

The stable oral care compositions of the present disclosure optionally comprise one or more active material(s), which is operable for the prevention or treatment of a condition or disorder of hard or soft tissue of the oral cavity, the prevention or treatment of a physiological disorder or condition, or to provide a cosmetic benefit.

Some embodiments of the present disclosure comprise a dental abrasive or combination of dental abrasive agents. As used herein, the term "abrasive" or "abrasive agent" also includes materials commonly referred to as "polishing agents." Any orally acceptable abrasive can be used, but typically, type, fineness (particle size) and amount of abrasive should be selected so that tooth enamel is not excessively abraded in normal use of the composition. Suitable abrasives include without limitation silica (in the form of silica gel, hydrated silica or precipitated silica), alumina, insoluble phosphates, calcium carbonate, resinous abrasives such as urea-formaldehyde condensation products and the like.

Among insoluble phosphates useful as abrasives are orthophosphates, polymetaphosphates and pyrophosphates. Illustrative examples are dicalcium orthophosphate dihydrate, calcium pyrophosphate, n-calcium pyrophosphate, tricalcium phosphate, calcium polymetaphosphate, tetrasodium pyrophosphate and insoluble sodium polymetaphosphate.

Average particle size of an abrasive, if present, is generally about 0.1 to about 30 $\mu$m for example about 1 to about 20 $\mu$m or about 5 to about 15 $\mu$m. In some embodiments, one or more abrasives are present in an amount of about 0.1 % to about 40% w/w. In some embodiments, the abrasive is calcium pyrophosphate. In some embodiments, the calcium pyrophosphate is present in an amount from about 5% to about 50% w/w.

In various embodiments of the present disclosure, the oral care composition comprises an anticalculus agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent is present in an amount of about 0.1% to about 30% w/w. In some embodiments, the stable oral care composition comprises a mixture of anticalculus agents. In some embodiments, tetrasodium pyrophosphate (TSPP) and sodium tripolyphosphate (STPP) are used as the anticalculus agents. In some embodiments, the anticalculus agent comprises about 1% to about 2% w/w TSPP, and about 0% to about 7% w/w STPP.

Another desirable component of the present compositions is a synthetic anionic polymeric polycarboxylate (SAPP), which acts as a stabilizer for the polyphosphate anti-tartar agent and may help to block access of painful or pain-causing materials, such as sugars, to the tooth nerves.

In some embodiments, the oral composition comprises a whitening agent. Any whitening agent known or developed in the art may be used in the present stable oral care composition. In various embodiments, the whitening agent is an oxidizing agent, a reducing agent or combinations thereof. In its broadest sense, "oxidizing agent" is intended to include those compounds which can accept an electron from another molecule in the environment of the oral cavity without having a deleterious or unacceptably harmful affect on the oral cavity in normal and accepted use.

Oxidizing agents suitable for use with the present stable oral care composition include peroxides, metal chlorites and persulfate. Exemplary peroxides include hydroperoxides, hydrogen peroxide, peroxides of alkali and alkaline earth metals, organic peroxy compounds, peroxy acids, pharmaceutically-acceptable salts thereof, and mixtures thereof.

Peroxides of alkali and alkaline earth metals include lithium peroxide, potassium peroxide, sodium peroxide, magnesium peroxide, calcium peroxide, barium peroxide, and mixtures thereof. Organic peroxy compounds include urea peroxide, glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as perborate salts of alkali and alkaline earth metals such as lithium, potassium, sodium, magnesium, calcium and barium, and mixtures thereof.

In some embodiments, the oxidizing agent comprises hydrogen peroxide. In some embodiments, the oxidizing agent comprises from 0.1 % to 50% w/w, such as from 0.1 % to 40% w/w, and more typically from 0.1% to 30% w/w, of the stable oral care composition. In other embodiments, the oxidizing agent consists essentially of hydrogen peroxide. In some embodiments, the hydrogen peroxide comprises from 0.1 % to 3% w/w, such as from 0.1 % to 2% w/w, and more typically about 1% to 4% w/w of the stable oral care composition.

In other embodiments, the whitening agent is a reducing agent. In its broadest sense, this term is intended to include those compounds which can donate an electron to another molecule in the environment of the oral cavity without having a deleterious or unacceptably harmful affect on the oral cavity in normal and accepted use. Synonyms for this term are preservatives or anti-oxidizing agents. There are numerous compounds which have been proven to be useful as reducing agents. A list of such compounds currently recognized for this purpose can be found in reference manuals and compendia covering pharmaceutical and oral care products. For example, *see* Martindale the Extra Pharmacopoeia, The Pharmaceutical Press, (London), Thirtieth Ed. James E. F. Reynolds Ed. (1993) beginning at page 1132-1139 (Martindale).

In so far as they meet the standards of oral acceptability, any compounds known in the art have the potential to be used in the stable oral care composition of the present disclosure, *e.g.*, vitamin C and its esters, vitamin E, the benzoates and hydroxybenzoates, butylated hydroxytoluene (BHT), butylated hydroxyanisole (BHA) and other reducing phenols, derivatives of dihydroxyquinoline, derivatives of polymerized 2,2,4- trimethyl-1,2-dihydroquinoline and alkyl gallate such as dodecyl gallate, ethyl gallate, octyl gallate and propyl gallate. In some embodiments, vitamin C, vitamin E, BHA, BHT, propyl gallate and combinations thereof are used.

In some embodiments, a solid whitening agent, such as sodium perborate, urea peroxide, calcium chlorite, barium chlorite, magnesium chlorite, lithium chlorite, sodium chlorite, potassium chlorite or mixtures thereof are used in the stable oral care composition described herein.

In some embodiments, the whitening agent is bound, for example, to a polymer, such as PVP (poly(N-vinylpyrrolidone). Suitable PVP complexes are disclosed, for example, in U.S. Patent No. 5,122,370.

In some embodiments, the stable oral care composition optionally comprises a source of fluoride ions. In some embodiments, the source of fluoride ions is selected from: fluoride, monofluorophosphate (MFP), and fluorosilicate salts. In some embodiments, one or more fluoride ion-releasing compounds are optionally present in an amount providing a total of 100 to 20,000 ppm, 200 to 5,000 ppm, or 500 to 2,500 ppm, fluoride ions.

The compositions also may include a stannous ion or a stannous ion source to mitigate calcium loss. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01% to about 10%, for example about 0.1% to about 7% or about 1% to about 5%.

The compositions of the present disclosure optionally comprise an antimicrobial (e.g., antibacterial) agent such as Cetylpyridinium chloride (CPC). An illustrative list of useful antibacterial agents is provided in U.S. Patent No. 5,776,435 to Gaffar et al.. One or more antimicrobial agents are optionally present in an antimicrobial effective total amount, typically about 0.05% to about 10%, for example about 0.1% to about 3% 0.075% to about 3%..

pH adjusting agents and the like may also be incorporated into the present oral care compositions. In some embodiments, the pH of the compositions is maintained in a range of from about 5 to about 10.5, such as from about 5 to about 9, such as from about 7 to about 9, such as from about 5 to about 7.5 or such as from about 5.5 to about 7. In some embodiments, for example oral care compositions containing calcium carbonate or insoluble phosphate abrasives, the pH of the compositions is maintained in a range from about 8.5 to 10.5.

Methods

In various embodiments, the present disclosure provides methods of preparing an oral care composition including mixing water into an orally acceptable vehicle to form a mixture (a) and mixing a colorant encapsulated in a biodegradable polylactic acid-based polymer into the mixture formed in (a) to form the oral care composition.

For example, in some embodiments desired thickeners may be combined with water and mixed to form a homogenous mixture. Sweeteners and other active ingredients, such as anticalculus agents, may then be added and mixed into the homogenous mixture followed by mixing in flavors and surfactants. The colorants encapsulated by the present biodegradable polymers may then be dispersed throughout the orally acceptable vehicle containing water and other ingredients.

The encapsulated colorants may vary by size and/or color and may be dispersed throughout the vehicle containing water, such that the stable oral care composition appears to contain speckles, speckled stripes, wavy stripes, straight stripes, spots, dots or other pattern formed by the encapsulated colorants. In other embodiments, the encapsulated colorants may be formulated and added in sufficient quantity such that the entire present oral care composition visually appears to be one solid color.

The oral care compositions of the invention can be used to promote oral health in a human or animal subject by contacting an oral surface with said oral care composition. As used herein, the phrase "promote oral health" encompasses preventing, curing, reversing, attenuating, alleviating, ameliorating, minimizing, suppressing or halting the deleterious effects of one or more oral conditions in a human or animal. The phrase "oral conditions" include but are not limited to plaque, tartar, stains, halitosis, gingivitis, periodontitis, and combinations thereof.

As used herein "animal subject" includes non-human mammals such as canines, felines, and horses. The stable oral care composition is contacted with an oral surface of the mammalian subject to thereby promote oral health in a highly efficacious manner.

In various embodiments, the stable oral care composition prepared in accordance with the present disclosure may be applied regularly to an oral surface, for example on a daily basis, at least one time daily for multiple days, or alternately every second or third day. In some embodiments, the stable oral care composition is applied to the oral surfaces from 1 to 3 times daily, for at least 2 weeks up to 8 weeks, from four months to three years, or more up to lifetime.

The examples and other embodiments described herein are exemplary and not intended to be limiting in describing the full scope of compositions and methods of this disclosure. Equivalent changes, modifications and variations of specific embodiments, materials, compositions and methods may be made within the scope of the present disclosure, with substantially similar results.

**EXAMPLES**

Example 1-Structured Mouthrinse

Three mouthrinse formulations, A, B and C were prepared by mixing the ingredients described in Table 1, below. Formulas A and B as shown in Table 1 were structured with a combination of xanthan (0.110 wt%, Formula A and 0.090 wt%, Formula B) and gellan gums (0.147 wt%, Formula A and 0.122 wt%, Formula B). Formula C did not contain the gum components. NaCl was used to attain a viscosity ranging from 255 to 595 (reported in Table 1 in centipoise, (cps), wherein 1 cps = 0.001Pa·s). 0.5 wt% of FD & C Blue No. 1 Aluminum Lake encapsulated in polylactic acid, approximately 230 μm in diameter, was added to each formulation. The encapsulated particles were obtained from Micro Powders, Inc., Tarrytown, NY.

The viscosity of the mouthrinse formulations were measured at 25°C at 1 rpm using a Brookfield Viscometer Model HADV-II + Pro, available from Brookfield Engineering Laboratories, Middleboro, MA. Due to the low viscosity of the formulation, a V73 spindle was used, which is sufficiently large to allow for greater accuracy of the viscosity. The spindle was rotated at pre-set rotations per minute (RPM). Torque (Torque%) was determined in terms of the % of its maximum (Tmax) value as specified for the instrument (Tmax = 1.437 mN·m for HADVII+Pro). Only those measurements with Torque% between 10 and 100% of Tmax were considered valid. The raw data (Torque% and RPM) were converted into shear stress (SS) and shear rate (SR) values using well known formulas for Couette geometry. The vane (spindle) was assumed to perform as its encompassing cylinder (i.e., it was assumed that the sample between the blades moves as a solid piece). Viscosity was determined as follows:

$$SR = SRC*RPM$$

$$SS = SF*Torque\%$$

where

$$SRC = (\pi/15)C/(1-x^2)$$

$$SF = 0.01*Tmax*C/(2\,\pi LR^2)$$

$$C = (1+x^2)/2,$$

where L is the vane blade length (meters) and R is the vane radius (meters); and x is the ratio of spindle diameter to the diameter of the vessel in which the measurement is performed (* denotes multiplication). Tmax is in N·m, SS is in Pascals and SR is in reciprocal seconds.

The viscosity in Table 1 is the SS/SR value at 1 RPM. In the test, RPM was swept from 0.5 to 200 in 20 steps in logarithmical mode, 10 sec per step.

The SS(SR) function was fitted with the Casson equation, $SS = (Y^n+(V0*SR)^n)^{1/n}$ where Y, V0 and n are fitting parameters. Only the monotonously increasing section of the curve bended upward (i.e., the one in which both first and second derivative of SS(SR) were positive, typically above 1 RPM) was fitted. "Yield stress" (YS) reported in Table 1 refers to the Y parameter.

As indicated in Table 1, microstructured mouthrinses exhibiting a YS value of at least 0.3 Pa (Formulas A and B) were effective in providing a homogenous suspension of particles in the continuous phase, despite the high density of encapsulated particles (approximately 1.26 g/cm$^3$) in comparison to the density of the continuous phase of the mouthrinse 1.0 g/cm$^3$. Accordingly, Formulas A and B as shown in Table 1 exhibit effective levels of gellan and xanthan materials to provide the necessary yield stress for homogenous suspension of encapsulated particles. In contrast, formula C, which is not structured with xanthan and gellan is not capable of prolonged suspension of PLA particles.

Surprisingly, the PLA speckles remained intact after three months of aging at controlled room temperature or under accelerated conditions (40°C and 75% humidity). Accordingly, the encapsulated colorants as described in the present disclosure result in stable compositions, even when the composition have a high water content.

Table 1. Mouthrinse Formulations with PLA Speckles

| Formula No. | A (wt%) | B (wt%) | C (wt%) |
|---|---|---|---|
| Demineralized water | 87.712 | 87.472 | 87.455 |
| Glycerin-USP Veg. Source | 4.900 | 4.900 | 4.900 |
| Propylene Glycol | 4.440 | 4.440 | 4.440 |
| Sodium Chloride | 0.866 | 0.290 | 0.866 |
| PLA Speckles with FD &C Blue 1 Aluminum Lake | 0.500 | 0.500 | 0.500 |
| Polysorbate 20 | 0.490 | 0.490 | 0.490 |
| Poloxomer 407 | 0.490 | 0.490 | 0.490 |
| Sodium Benzoate | 0.490 | 0.490 | 0.490 |

(continued)

| Formula No. | A (wt%) | B (wt%) | C (wt%) |
|---|---|---|---|
| 85% Syrupy Phosphoric Acid | 0.390 | 0.390 | 0.390 |
| Low Acyl Gellan Gum | 0.147 | 0.122 | 0.000 |
| Flavor | 0.140 | 0.140 | 0.140 |
| Xanthan Gum | 0.110 | 0.090 | 0.000 |
| Cetylpyridinium Chloride | 0.075 | 0.075 | 0.075 |
| Sodium Fluoride | 0.050 | 0.050 | 0.050 |
| Sodium Saccharin | 0.050 | 0.050 | 0.050 |
| Sucralose | 0.020 | 0.020 | 0.020 |
| FD & C Blue No. 1 (C142090) | 0.001 | 0.001 | 0.001 |
| Viscosity/YS(Pa) at Room Temperature, 1 rpm, V73 spindle | | | |
| Initial | 520/0.44 | 255/0.30 | 405/0.00 |
| 3 Months | 595/0.49 | 390/0.32 | 435/0.00 |
| Physical Stability after 3 months at controlled room temperature | | | |
| | Pass PLA speckles homogenous and intact | Pass PLA speckles homogenous and intact | Fail PLA speckles intact but settled at bottom |

Example 2. Aqueous Liquigel Toothpaste

An aqueous liquigel toothpaste with both mouthrinse breath-freshening and toothpaste cleaning components was prepared by mixing the ingredients described in Table 2. 0.3 wt% PLA speckles were added to the formulation. The viscosity and YS values were determined as described above.

As indicated in Table 2, the finished viscosity of the formulation ranged from 120,000 to 270,000 cps. The pH ranged from 7.0 to 9.0.

The PLA speckles have a specific gravity value similar to the continuous phase of the toothpaste. A homogenous distribution of the PLA speckles was maintained during the three month storage period. Without being limited by theory, the homogenous distribution of the speckles is provided by the low concentration of silica thickener and xanthan, which contributes to the static yield stress (YS) of the formulation. The PLA speckles remained intact in this formulation after three months of aging under accelerating conditions.

Table 2. Liquigel Toothpaste with PLA Particles

| Formula No. | D (wt%) |
|---|---|
| Sorbitol, N.C. (70%. AI) | 33.000 |
| Demineralized Water | 26.517 |
| Glycerin-USP Veg. Source | 12.000 |
| Ppt. Silica Abrasive | 11.000 |
| High Cleaning Ppt. Silica | 10.000 |
| Ppt. Silica Thickener | 1.500 |
| Sodium Lauryl Sulfate | 1.500 |
| Cocamidpropyl Betaine (30%, AI) | 1.250 |
| Flavor | 1.100 |

(continued)

| Formula No. | D (wt%) |
|---|---|
| Tetrasodium Pyrophosphate | 0.500 |
| Sodium Saccharin | 0.400 |
| PLA Speckles with FD &C Blue 1 Aluminum Lake | 0.300 |
| Na carboxymethylcellulose Type 7/500 T | 0.260 |
| Titanium Dioxide | 0.250 |
| Sodium Fluoride-USP | 0.243 |
| Xanthan Gum | 0.180 |
| Viscosity (x10,000 cps)/YS (Pa) at 25°C/60% relative humidity, 1 rpm V73 spindle | |
| Initial | 12/27 |
| 3 months | 27/35 |
| Specific Gravity | |
| | 1.29 |
| Physical Description | |
| | White viscous liquid with blue speckles |

Example 3. Aqueous Toothpastes and Gels

PLA speckles with white (TiO$_2$), blue (FD &C Blue Aluminum Lake 1, green (combination of FD & C Blue Aluminum Lake D &C yellow 5 lake and red (FD & C 40/Lake) colors were formulated in aqueous paste or gel formulations to assess the robustness and/or compatibility of these formulations with different ingredients. Formulations E-H as shown in Table 3 were prepared by combining the specified ingredients. Viscosity and YS values were determined as described above, except that a smaller (V74) vane spindle was used due to the higher viscosity of the formulations to ensure that maximum torque capability of the instrument was not exceeded.

As indicated in Table 3, the finished viscosity of the formulations ranged from 226,000 to 554,000 cps and the pH ranged from 7.33 to 8.05. As also indicated in the Table, three month aging evaluations at controlled room temperature and accelerated high temperature conditions resulted in acceptable chemical and physical stability of the finished formulations. In particular, color migration from the PLA encapsulated speckles into the toothpaste vehicle was not visually observed. Further, homogenous distribution of the PLA speckles was maintained during the storage period. This homogeneity was likely due to the presence of xanthan, carboxymethylcellulose, silica thickener and/or methyl vinyl ether/maleic anhydride copolymer, which provide adequate viscosity and static yield stress properties in the formulations.

Table 3 Aqueous Toothpastes/Gels with PLA Particles

| Formula No. | E (wt%) | F (wt%) | G (wt%) | H (wt%) |
|---|---|---|---|---|
| Sorbitol, N.C. (70%. AI) | 34.700 | 34.700 | 16.000 | 0.000 |
| Demineralized Water | 28.606 | 28.107 | 18.748 | 28.202 |
| Glycerin-USP Veg. Source | 0.000 | 0.000 | 16.500 | 26.500 |
| Synthetic Ppt. Silica-Abrasive | 11.250 | 11.250 | 10.000 | 10.000 |
| High Cleaning Silica | 10.000 | 10.000 | 10.000 | 0.000 |
| Potassium Nitrate, USP | 0.000 | 0.000 | 5.000 | 5.000 |
| Polyethylene Glycol 600 | 3.000 | 3.000 | 3.000 | 3.000 |
| Ppt. Silica Thickener | 2.750 | 2.750 | 1.750 | 8.000 |
| Cocamidopropyl Betaine (30%, AI) | 2.500 | 2.500 | 0.000 | 0.000 |
| Poloxomer 407, USP | 2.000 | 2.000 | 0.000 | 0.000 |
| Zinc Citrate, Trihydrate | 0.000 | 0.000 | 2.000 | 0.000 |
| Sodium Lauryl Sulfate | 1.450 | 1.450 | 1.500 | 0.435 |
| PVM/MA Copolymer (16.5% AI) | 0.000 | 0.000 | 9.090 | 11.800 |
| Flavor | 1.450 | 1.450 | 1.300 | 1.100 |
| Sodium Hydroxide, 50% Solution | 0.000 | 0.000 | 1.000 | 1.450 |
| Sodium Monofluorophosphate | 0.000 | 0.000 | 1.140 | 0.000 |
| Sodium Phosphate, Tribasic, 12-Hydrate | 0.000 | 0.000 | 0.000 | 1.000 |
| Xanthan Gum | 0.850 | 0.850 | 0.400 | 0.170 |
| Tetrasodium Pyrophosphate | 0.500 | 0.500 | 2.000 | 0.000 |
| Titanium Dioxide, FD & C | 0.000 | 0.500 | 0.000 | 1.000 |

| | | | | |
|---|---|---|---|---|
| Poloxomer 407 | 0.000 | 0.000 | 0.000 | 1.000 |
| Sodium CMC-Type 12 | 0.000 | 0.000 | 0.600 | 0.400 |
| Sodium Saccharin | 0.400 | 0.400 | 0.700 | 0.400 |
| Titanium Dioxide Coated Mica | 0.000 | 0.000 | 0.150 | 0.000 |
| White PLA Speckles | 0.300 | 0.000 | 0.000 | 0.000 |
| Blue PLA Speckles | 0.000 | 0.300 | 0.000 | 0.000 |
| Green PLA Speckles | 0.000 | 0.000 | 0.120 | 0.000 |
| Red PLA Speckles | 0.000 | 0.000 | 0.000 | 0.300 |
| Sodium Fluoride | 0.243 | 0.243 | 0.000 | 0.243 |
| FD &C Blue #1 (CI 42090) | 0.001 | 0.000 | 0.001 | 0.000 |
| D & C Yellow No. 10 (CI 47005) | 0.000 | 0.000 | 0.001 | 0.000 |
| Viscosity (x10,000 cps)/YS (Pa) at Room Temperature, 1 rpm, V74 spindle | | | | |
| Initial | 24.9/37 | 22.6 / 55 | 24.3/120 | 47.5/155 |
| 3 months | 18.6/85 | 26.7/92 | 27.1/197 | 55.4/320 |
| pH (10% solution) | | | | |
| Initial | 7.83 | 7.78 | 7.44 | 7.96 |
| 3 months at 25°C/60% relative humidity | 7.79 | 7.79 | 7.38 | 7.94 |
| 3 months at 40°C/75% relative humidity | 7.74 | 7.71 | 7.33 | 8.05 |
| Soluble F- ppm | | | | |
| Initial | 1020 | 1050 | 1550 | 1110 |
| 3 months at 25°C/60% relative humidity | 1000 | 1020 | 1397 | 1060 |
| 3 months at 40°C/75% relative humidity | 960 | 990 | 1250 | 940 |
| Specific Gravity | | | | |
| | 1.29 | 1.29 | 1.37 | 1.27 |
| Physical Description | | | | |
| | Blue Gel with White Speckles | White Paste with Blue Speckles | Green Gel with Green Speckles & Mica | White Paste with Red Speckles |

Example 4. Non-Aqueous Toothpastes with PLA Speckles

A nonaqueous toothpaste containing a whitening agent, hydrogen peroxide, was formulated by combining the ingredients specified in Table 4, below. The viscosity and YS values were determined as described in Example 3, above.

Formulation "I" as indicated in the Table, produced a semi-translucent gel with a blue speckling aesthetic. The second formulation, Formulation "J", produced a white paste with a blue speckling aesthetic. The finished viscosity ranged from 382,000 to 683,000 cps. The pH was between 7.9 and 8.9.

The formulations demonstrated acceptable chemical and physical stability after three months of aging at 25°C at 60% relative humidity and under accelerated aging conditions (40°C at 75% relative humidity). Further, only minimal color migration was visually observed from the PLA encapsulated colorants into the toothpaste vehicle. Accordingly, the PLA was stable even in the presence of 1% hydrogen peroxide. While only the FD & C blue No. 1 aluminum lake colorant was evaluated, PLA particles containing other lake and pigment colors such as D &C Yellow 5 aluminum Lake, FD &C Red 40 Aluminum Lake and titanium oxide are also expected to be suitably stable due to the encapsulation within the PLA matrix.

Table 4. Non-Aqueous Toothpastes with PLA Particles

| Formula No. | I (wt%) | J (wt%) |
|---|---|---|
| Glycerin-USP Veg. Source | 54.327 | 0.000 |
| Ethylene Oxide Propylene Oxide Copolymer | 0.000 | 27.000 |
| Propylene Glycol/Polypropylene Glycol Copolymer | 0.000 | 25.000 |
| High Cleaning Ppt. Silica | 20.000 | 0.000 |
| Calcium Pyrophosphate | 0.000 | 19.000 |
| Polyethylene Glycol 600 | 10.000 | 0.000 |
| Sodium Tripolyphosphate, Anhydrous | 0.000 | 7.000 |
| Propylene Glycol | 0.000 | 6.500 |
| Cross-linked PVP complexed with Hydrogen Peroxide | 5.500 | 5.500 |
| Ppt. Silica Thickener | 2.500 | 0.000 |
| Flavor | 2.200 | 2.210 |
| Sodium Lauryl Sulfate | 2.000 | 2.000 |
| Tetrasodium Pyrophosphate | 2.000 | 2.000 |
| Fumed Silica | 0.000 | 1.500 |
| Sodium Saccharin | 0.000 | 0.800 |
| Na MFP-USP | 0.000 | 0.760 |
| Carbomer 974P | 0.500 | 0.000 |
| PLA Speckles with FD &C Blue 1 Aluminum Lake | 0.500 | 0.500 |
| Sodium Fluoride | 0.243 | 0.000 |
| Syrupy Phosphoric Acid-Food Grade | 0.000 | 0.200 |
| Sucralose | 0.200 | 0.000 |
| Butylated Hydroxytoluene FCC Grade | 0.030 | 0.030 |
| Viscosity (x10,000 cps)/YS (Pa) at Room Temperature, 1 rpm, V74 spindle | | |

| | I | J |
|---|---|---|
| Initial | 45.3 / 170 | 38.2 / 290 |
| 3 months | 68.3 / 255 | 55.4 / 319 |
| Specific Gravity | | |
| | 1.35 | 1.31 |
| Hydrogen Peroxide (%) at 25°C/60% Relative Humidity | | |
| Initial | 1.02 | 1.05 |
| 3 months | 0.85 | 0.98 |
| Soluble F-, ppm | | |
| Initial | 1092 | 1123 |
| 3 months at 25°C/60% relative humidity | 1004 | 1036 |
| 3 months at 40°C/75% relative humidity | 975 | 988 |
| Physical Description | Semi-translucent Gel with Blue Speckles | White Paste with Blue Speckles |

**Claims**

1. A stable oral care composition comprising:

   an orally acceptable vehicle, and
   a colorant encapsulated by a biodegradable polylactic acid-based polymer.

2. The stable oral care composition of claim 1, wherein the oral care composition comprises at least 10% by weight of water.

3. The stable oral care composition of claim 1, wherein the oral care composition comprises at least 25% by weight of water.

4. The stable oral care composition of claim 1, wherein the oral care composition comprises at least 75% by weight of water.

5. The stable oral care composition of claim 1, wherein the biodegradable polylactic acid-based polymer is a polylactic acid copolymer having a 75:25 weight ratio of polylactic acid: polyglycolide with a molecular weight of 76,000-116,000 daltons.

6. The stable oral care composition of claim 1, wherein the colorant is selected from the group consisting of a lake, a dye, a pigment and a combination thereof.

7. The stable oral care composition of claim 6, wherein the colorant is a lake selected from the group consisting of FD & C Blue Aluminum No. 1 Lake, FD & C Blue Aluminum No. 2 Lake, FD & C Red Aluminum No. 3 Lake, FD & C Yellow Aluminum No. 5 Lake, FD & C Aluminum Yellow No. 6 Aluminum Lake and combinations thereof.

8. The stable oral care composition of claim 6, wherein the colorant is a dye selected from the group consisting of FD&C Red No. 3, Food Red 17, Food Yellow 13, FD&C Yellow No. 5, FD&C Yellow No. 6, FD&C Green No. 3, FD&C Blue No. L FD&C Blue No. 2 and combinations thereof.

9. The stable oral care composition of claim 1, wherein the colorant encapsulated by the biodegradable polylactic acid-based polymer causes the composition to have a speckled appearance.

10. A method of preparing an oral care composition comprising:

    (a) mixing water into an orally acceptable vehicle to form a mixture; and
    (b) dispersing a colorant encapsulated in a biodegradable polylactic acid-based polymer into the mixture formed in (a) to form a stable oral care composition.

**Patentansprüche**

1. Stabile Mundpflegezusammensetzung, umfassend:

   ein oral unbedenkliches Vehikel und
   ein Farbmittel, das von einem biologisch abbaubaren Polymer auf Polymilchsäurebasis eingekapselt ist.

2. Stabile Mundpflegezusammensetzung nach Anspruch 1, wobei die Mundpflegezusammensetzung mindestens 10 Gew.-% Wasser umfasst.

3. Stabile Mundpflegezusammensetzung nach Anspruch 1, wobei die Mundpflegezusammensetzung mindestens 25 Gew.-% Wasser umfasst.

4. Stabile Mundpflegezusammensetzung nach Anspruch 1, wobei die Mundpflegezusammensetzung mindestens 75 Gew.-% Wasser umfasst.

5. Stabile Mundpflegezusammensetzung nach Anspruch 1, wobei das biologisch abbaubare Polymer auf Polymilchsäurebasis ein Polymilchsäure-Copolymer mit einem Gewichtsverhältnis von 75:25 von Polymilchsäure:Polyglykolid mit einem Molekulargewicht von 76.000-116.000 Dalton ist.

6. Stabile Mundpflegezusammensetzung nach Anspruch 1, wobei das Farbmittel aus der Gruppe bestehend aus einem Farblack, einem Farbstoff, einem Pigment und einer Kombination davon ausgewählt ist.

7. Stabile Mundpflegezusammensetzung nach Anspruch 6, wobei das Farbmittel ein Farblack ist, der aus der Gruppe bestehend aus Farblack FD & C Blue Aluminum No. 1, Farblack FD & C Blue Aluminum No. 2, Farblack FD & C Red Aluminum No. 3, Farblack FD & C Yellow Aluminum No. 5, Farblack FD & C Aluminum Yellow No. 6 Aluminum und Kombinationen davon ausgewählt ist.

8. Stabile Mundpflegezusammensetzung nach Anspruch 6, wobei das Farbmittel ein Farbstoff ist, der aus der Gruppe bestehend aus FD&C Red No. 3, Food Red 17, Food Yellow 13, FD&C Yellow No. 5, FD&C Yellow No. 6, FD&C Green No. 3, FD&C Blue No. L FD&C Blue No. 2 und Kombinationen davon ausgewählt ist.

9. Stabile Mundpflegezusammensetzung nach Anspruch 1, wobei das durch das biologisch abbaubare Polymer auf Polymilchsäurebasis eingekapselte Farbmittel bewirkt, dass die Zusammensetzung ein gesprenkeltes Aussehen hat.

10. Verfahren zum Herstellen einer Mundpflegezusammensetzung, umfassend:

(a) Mischen von Wasser in ein oral unbedenkliches Vehikel zur Bildung einer Mischung, und
(b) Dispergieren eines in einem biologisch abbaubaren Polymer auf Polymilchsäurebasis eingekapselten Farbmittels in der in (a) gebildeten Mischung, um eine stabile Mundpflegezusammensetzung zu bilden.

**Revendications**

1. Composition d'hygiène buccale stable, comprenant :

un excipient oralement acceptable, et
un colorant encapsulé dans un polymère biodégradable à base d'acide polylactique.

2. Composition d'hygiène buccale stable selon la revendication 1, dans laquelle la composition d'hygiène buccale comprend au moins 10 % en poids d'eau.

3. Composition d'hygiène buccale stable selon la revendication 1, dans laquelle la composition d'hygiène buccale comprend au moins 25 % en poids d'eau.

4. Composition d'hygiène buccale stable selon la revendication 1, dans laquelle la composition d'hygiène buccale comprend au moins 75 % en poids d'eau.

5. Composition d'hygiène buccale stable selon la revendication 1, dans laquelle le polymère biodégradable à base d'acide polylactique est un copolymère d'acide polylactique ayant un rapport pondéral de 75:25 d'acide polylactique: polyglycolide ayant un poids moléculaire de 76 000 à 116 000 daltons.

6. Composition d'hygiène buccale stable selon la revendication 1, dans laquelle le colorant est choisi dans le groupe constitué d'une laque, d'un colorant, d'un pigment et d'une combinaison de ceux-ci.

7. Composition d'hygiène buccale stable selon la revendication 6, dans laquelle le colorant est une laque choisie dans le groupe constitué de la laque FD & C bleu aluminium n°1, laque FD & C bleu aluminium n°2, laque FD & C rouge aluminium n°3, laque FD & C jaune aluminium n°5, laque FD & C aluminium jaune n°6 et de combinaisons de ceux-ci.

8. Composition d'hygiène buccale stable selon la revendication 6, dans laquelle le colorant est un colorant choisi dans le groupe constitué de FD&C rouge n°3, rouge alimentaire 17, jaune alimentaire 13, FD&C jaune n°5, FD&C jaune n°6, FD&C vert n°3, FD&C bleu n°L FD&C bleu n°2 et de combinaisons de ceux-ci.

9. Composition d'hygiène buccale stable selon la revendication 1, dans laquelle le colorant encapsulé dans le polymère biodégradable à base d'acide polylactique donne à la composition un aspect moucheté.

10. Procédé de préparation d'une composition d'hygiène buccale, comprenant les étapes consistant à :

(a) mélanger l'eau dans un excipient oralement acceptable pour former un mélange ; et
(b) disperser un colorant encapsulé dans un polymère biodégradable à base d'acide polylactique dans le mélange formé en (a) pour former une composition d'hygiène buccale stable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014088534 PCT
- US 5122370 A

- US 5776435 A, Gaffar

### Non-patent literature cited in the description

- Kirk-Othmer Encyclopedia of Chemical Technology. vol. 6, 561-596
- Kirk-Othmer Encyclopedia of Chemical Technology. 1981, vol. 125, 470-493

- Martindale the Extra Pharmacopoeia. The Pharmaceutical Press, 1993, 1132-1139